Europäisches Patentamt

European Patent Office

Office européen des brevets

(19)

(11) **EP 0 855 912 B1**

(12) **FASCICULE DE BREVET EUROPEEN**

(45) Date de publication et mention
de la délivrance du brevet:
**05.12.2001 Bulletin 2001/49**

(21) Numéro de dépôt: **96934916.6**

(22) Date de dépôt: **15.10.1996**

(51) Int Cl.$^7$: **A61K 33/00**

(86) Numéro de dépôt international:
**PCT/FR96/01610**

(87) Numéro de publication internationale:
**WO 97/15311 (01.05.1997 Gazette 1997/19)**

(54) **COMPOSITION A BASE DE MONOXYDE D'AZOTE EN TANT QUE MEDICAMENT**

PHARMAZEUTISCHE ZUSAMMENSETZUNG VON STICKSTOFFMONOXID

NITROGEN MONOXIDE COMPOSITION FOR USE AS A DRUG

(84) Etats contractants désignés:
**AT BE CH DE DK ES FI FR GB GR IT LI NL PT SE**

(30) Priorité: **20.10.1995 FR 9512345**

(43) Date de publication de la demande:
**05.08.1998 Bulletin 1998/32**

(73) Titulaire: **L'AIR LIQUIDE, SOCIETE ANONYME POUR**
**L'ETUDE ET L'EXPLOITATION DES PROCEDES**
**GEORGES CLAUDE**
**75321 Paris Cédex 07 (FR)**

(72) Inventeurs:
• **BRIEND, Robert**
  **F-78340 Les Clayes-sous-Bois (FR)**

• **RENAUDIN, Marie-Hélène**
  **F-75015 Paris (FR)**

(74) Mandataire: **Le Moenner, Gabriel et al**
**L'AIR LIQUIDE, Société Anonyme**
**pour l'étude et l'exploitation des procédés**
**Georges Claude**
**75, Quai d'Orsay**
**75007 Paris (FR)**

(56) Documents cités:
**WO-A-92/10228        WO-A-94/00180**
**WO-A-94/22499**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne l'utilisation d'une composition gazeuse stable à base de monoxyde d'azote (NO) et de dioxyde de carbone ($CO_2$) pour la fabrication d'un médicament gazeux destiné au traitement ou à la prophylaxie par voie intra-vasculaire de l'ischémie, et, en particulier de l'embolie.

Le monoxyde d'azote est produit naturellement chez le mammifère par une enzyme, la NO-synthase, laquelle est exprimée de façon constitutive dans les cellules endothéliales, dans les plaquettes et dans le système nerveux central et périphérique. Une autre forme de NO-synthase calcium-indépendante, peut être induite par différents stimuli (notamment des liposaccharides) dans de nombreuses cellules telles que les macrophages, les lymphocytes, les cellules myocardiques, les cellules endothéliales et les cellules musculaires lisses.

Le monoxyde d'azote est un messager biologique important chez les mammifères et cette molécule joue un rôle déterminant dans le contrôle local de l'hémodynamique.

On a pu mettre en évidence la libération de NO par les cellules endothéliales en cas de variations du débit sanguin. Le monoxyde d'azote apparaît notamment comme une composante majeure de l'adaptation physiologique du diamètre vasculaire à la perfusion sanguine : ainsi au niveau coronaire, l'hypérémie réactive est atténuée de façon notoire. Inversement, une augmentation chronique du débit sanguin produite par une fistule artérioveineuse augmente les relaxations dépendantes de l'endothélium.

La capacité du monoxyde d'azote produit au niveau de la paroi vasculaire et dans les tissus avoisinants à réguler de façon précise le tonus vasculaire par adaptation du débit sanguin est remarquable. De même, on a émis l'hypothèse que le NO libéré lors de l'activité neuronale pourrait réguler le tonus de la microcirculation cérébrale, couplant ainsi l'activité et le débit sanguin cérébral. On rappellera également le rôle de NO dans la régulation de la prolifération du muscle lisse vasculaire laquelle est un facteur déterminant de la compliance vasculaire.

Le monoxyde d'azote contrôle par ailleurs la perméabilité veinulaire post-capillaire.

Le monoxyde d'azote participe aussi à des mécanismes de régulation hormonale, au niveau du rein en inhibant la libération de rénine, au niveau cardio-vasculaire, en antagonisant la libération du facteur natriurétique (ANF).

Enfin, in vivo, l'activation plaquettaire est sous le contrôle permanent du monoxyde d'azote endothélial, et dans un moindre degré sous celui de la propre NO synthase plaquettaire. Lors de l'agrégation, les plaquettes libèrent des nucléotides (ATP, ADP), de la sérotonine, du PAF, du thromboxane A2 et de la vasopressine; elles peuvent aussi initier la cascade de la coagulation en libérant de la thrombine. En réponse à l'ATP, l'ADP, la sérotonine, le PAF et la thrombine, les cellules endothéliales libèrent du NO et de la prostacycline qui agissent en synergie pour prévenir et contrer le processus d'agrégation plaquettaire.

La diminution anormale du taux de monoxyde d'azote observée dans le cas de nombreuses pathologies semble confirmer l'importance du rôle joué par celui-ci dans l'organisme. Une telle diminution est caractéristique de l'hypertension, de l'hypercholestérolémie, de l'athérosclérose et du diabète.

De même, une réduction très précoce de la libération basale de NO serait à l'origine des troubles liés à la reperfusion de territoires ischémiés, tels que la thrombose coronaire et le vasospasme.

Sur la base de ces diverses constatations, divers agents vasodilatateurs ont été mis au point à ce jour : ces substances connues sous la désignation de nitrovasodilatateurs produisent du NO in vivo et suppléent ainsi à un défaut de NO endogène. On peut citer, par exemple, la molsidomine ou le nitroprussiate de sodium, lesquels permettent de prévenir les phénomènes d'adhésion et d'agrégation plaquettaire.

De façon à pallier une production insuffisante de NO, on a, de même, proposé l'administration de L-arginine ou d'analogues de la L-arginine, la L-arginine intervenant directement dans la biosynthèse du monoxyde d'azote, en tant que substrat de la NO-synthase.

Au vu de la contribution significative du monoxyde d'azote au maintien d'une basse pression au niveau de la circulation pulmonaire et de l'importance de l'effet vasodilatateur local résultant, on a, par ailleurs, suggéré l'administration directe de NO par voie inhalée dans le traitement de l'hypertension artérielle pulmonaire aiguë. Les nombreuses recherches réalisées en ce sens ont démontré l'efficacité thérapeutique d'un mélange gazeux de monoxyde d'azote et d'azote inhalé à des doses comprises entre 1 et 20 ppm de NO sur des patients souffrant de détresse respiratoire aiguë : une réduction de l'hypertension artérielle pulmonaire éventuellement accompagnée d'une amélioration des rapports ventilation-perfusion par baisse du shunt intrapulmonaire sont en effet observées.

Néanmoins, ce type d'administration par voie inhalée présente l'inconvénient majeur d'entraîner la formation de $NO_2$ par réaction du NO inhalé avec l'oxygène présent au niveau des alvéoles pulmonaires. Or, le dioxyde d'azote est une substance hautement toxique pour le poumon. Un autre désavantage de la méthode est lié à la nécessité de réaliser le mélange constitué de monoxyde d'azote, d'azote et d'oxygène de façon extemporanée, le temps de contact entre NO et $O_2$ devant être le plus court possible.

De plus, l'administration par voie inhalée limite de toute évidence l'action du monoxyde d'azote exogène aux zones ventilées du poumon.

Une injection intra-vasculaire du mélange utilisé constitué d'azote et de NO n'est en effet pas envisageable dans la mesure où elle entraînerait l'embolie gazeuse.

Par ailleurs, on connaît aussi le document WO-A-9400180 qui divulgue l'administration de monoxyde d'azote pour réduire les contractions des muscles lisses utérins ou de la vessie et l'administration du NO à l'état gazeux éventuellement avec du $CO_2$ dans un fluide biologique qui n'est pas du sang.

En outre, le document WO-A-9210228 divulgue l'inhalation de NO dans le traitement de l'asthme et le document WO-A-9422499 enseigne l'administration de composés comprenant du NO par inhalation dans le traitement de l'embolie et de l'embolie pulmonaire.

c'est dans ce contexte que la présente invention a été réalisée.

L'invention concerne alors l'utilisation d'une composition gazeuse contenant du monoxyde d'azote (NO) et du dioxyde de carbone ($CO_2$) pour la fabrication d'un médicament gazeux destiné au traitement ou à la prophylaxie par voie intra-vasculaire de l'ischémie, chez l'homme ou l'animal.

Plus précisément, l'invention concerne l'utilisation d'une composition gazeuse contenant du monoxyde d'azote (NO) et du dioxyde de carbone($CO_2$) pour la fabrication d'un médicament gazeux destiné au traitement ou à la prophylaxie par voie intra-vasculaire de l'embolie, chez l'homme ou l'animal.

Dans le cadre de la présente invention :

- le terme ischémie désigne un arrêt total ou partiel de la circulation sanguine dans une zone localisée de l'organisme humain ou animal ;
- et le terme embolie désigne, quant à lui, une oblitération d'un vaisseau sanguin par un caillot ou un corps étranger véhiculé par le sang jusqu'au lieu où le calibre du vaisseau sanguin est insuffisant pour permettre son passage.

L'embolie est donc une forme particulière d'ischémie.

Le médicament gazeux de l'invention est, de préférence, constituée du seul mélange de gaz carbonique et de monoxyde d'azote; toutefois, l'addition d'au moins un gaz dans le groupe formé par le xénon, le krypton, le protoxyde d'azote et leurs mélanges, audit mélange de $CO_2$ et de NO est également envisageable dans le cadre de l'invention.

La concentration de NO dans le mélange gazeux NO + $CO_2$ est une concentration efficace, de préférence comprise entre 1 et 100 ppm. Une concentration inférieure à 1 ppm n'est pas souhaitable dans la mesure où une concentration minimale de 2 nM de NO est détectée dans le plasma sanguin d'un mammifère sain. Une concentration supérieure à 100 ppm entraîne une disparition progressive de l'effet thérapeutique. Les raisons du peu d'activité observée à de telles concentrations sont encore inconnues, mais pourraient être liées à une toxicité locale ou éventuellement à la saturation des récepteurs du monoxyde d'azote.

Selon un mode de réalisation préféré, la concentration de NO dans le mélange médicamenteux est comprise entre 15 et 30 ppm.

La stabilité du médicament gazeux de l'invention permet son stockage sous pression dans des conditionnements classiques du type bouteilles en acier ou alliage léger à base d'aluminium.

De façon à éviter tout risque de contamination, on optera avantageusement pour des bouteilles en alliage léger à base d'aluminium. Les conditions de stockage préférées assurant une stabilité supérieure à 2 ans sont une température comprise entre 15 et 30°C, de préférence 20 et 25°C, et une pression comprise entre 20 et 30 bars.

Le médicament gazeux de l'invention est administrable par voie intra-vasculaire, sans risque d'embolie gazeuse, par exemple par voie intra-artérielle, par voie intra-cardiaque ou par voie intra-veineuse.

Dans le cas d'une administration intra-vasculaire, on aura préférablement recours à un cathéter introduit au niveau du vaisseau sanguin traité. Les cathéters utilisables à cet effet sont ceux habituellement employés dans la technique. La nature du matériau formant le cathéter n'est pas critique en soi; on recommande néanmoins un matériau souple, tel que le silastène. Son implantation dans le vaisseau sanguin est réalisée de façon connue en soi, par exemple, après anesthésie locale et incision. Par commodité le cathéter peut être maintenu par ligature à la suite de quoi l'incision peut être refermée ou non.

Lors de l'injection, on prendra les précautions nécessaires habituelles de façon à éviter l'agrégation plaquettaire et la coagulation au niveau de l'orifice.

En ce sens, on pourra avantageusement procéder à l'administration d'une solution physiologique héparinée avant et après injection du médicament gazeux de l'invention.

Concernant le débit d'injection, l'homme du métier pourra facilement le déterminer, étant entendu qu'une solubilisation immédiate du médicament gazeux au fur et à mesure de son administration est des plus souhaitables : ce faisant, on évitera la formation de bulles de gaz. Chez l'homme un débit inférieur à 20 ml/mn et, de préférence, inférieur à 10 ml/mn, mieux encore 8 ml/mn conduira à une efficacité optimale. Ces chiffres n'entendent cependant nullement limiter l'invention. Il est clair en effet, que le débit d'injection est fonction du débit sanguin du vaisseau traité.

La quantité de composition médicamenteuse devant être administrée dépend, quant à elle, de l'âge du patient, de la gravité de l'affection dont il souffre et de la concentration en NO de la composition gazeuse injectée.

Selon l'invention, une intervention au niveau d'un vaisseau artériel serait préférée. En effet, on a pu constater que dans les artères, la concentration en oxyhémoglobine est telle que la fixation de monoxyde d'azote sur l'hémoglobine est nettement défavorisée. De ce fait, la formation de méthémoglobine par liaison du monoxyde d'azote avec l'hémoglobine n'aurait lieu qu'en quantité insuffisante pour provoquer une éventuelle inhibition de l'effet thérapeutique du monoxyde d'azote et notamment de son effet vasodilatateur et anti-agrégant plaquettaire.

De récents travaux ont montré que le monoxyde d'azote peut aussi réagir avec des protéines, telle l'albumine, pour former des S-nitrosothiols, dont la durée de vie est plus longue que celle du NO libre. Ces espèces présentant une activité biologique comparable à celle du NO libre, on comprend que l'efficacité des compositions médicamenteuses de l'invention ne s'en trouve pas diminuée.

Les compositions médicamenteuses de l'invention conviennent parfaitement au traitement de l'ischémie et de l'embolie lorsque administrées par injection intra-vasculaire: intra-artérielle, intra-cardiaque ou intra-veineuse.

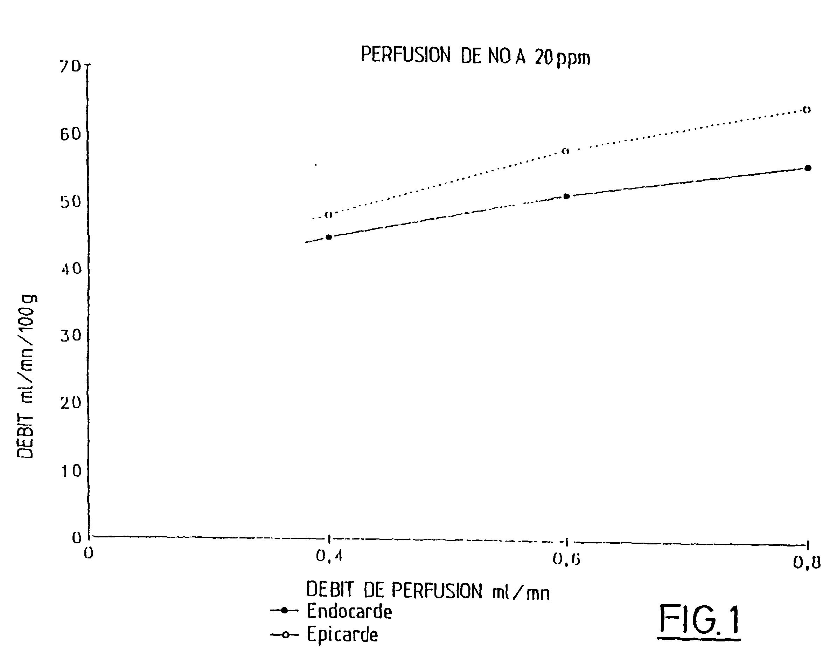

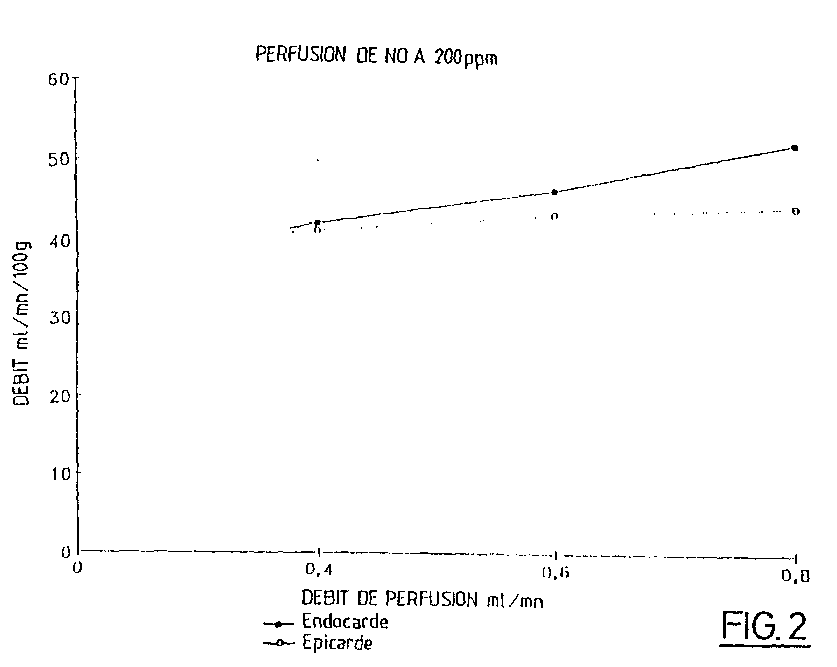

Les exemples 1 à 3 qui suivent illustrent la stabilité des compositions de l'invention, ainsi que leur utilité thérapeutique, en référence aux Figures 1 et 2 annexées.

EXEMPLE 1

Divers mélanges de monoxyde d'azote et de gaz carbonique ont été préparés et conditionnés dans des bouteilles de type B5 en alliage léger, à base d'aluminium, commercialisées par S.M. GERZAT sous une pression de 24 bars. Dans ces mélanges, la concentration initiale du monoxyde d'azote a été fixée à 20 ppm. La stabilité de la concentration en NO a été étudiée pour trois bouteilles conservées à température ambiante sur une période de 16 mois.

La valeur de la concentration en NO est mesurée par un analyseur à chimiluminescence dans la gamme de 0 à 100 ppm, calibré avant chaque mesure à l'aide d'un mélange étalon $NO/N_2$ à 90 ppm. L'analyseur à chimiluminescence utilisé est le TOPAZE 2020 fabriqué par COSMA.

A l'issue d'une période de 16 mois, aucune décomposition du monoxyde d'azote dans les mélanges $NO/CO_2$ conservés à température ambiante n'a pu être détectée. De fait, les seules fluctuations mesurées dans la valeur de la concentration en NO restent inférieures à la précision de l'analyse.

Ces résultats confirment la stabilité des compositions médicamenteuses de l'invention.

EXEMPLE 2

L'utilité thérapeutique des compositions médicamenteuses de l'invention a été démontrée sur un modèle expérimental. Plus précisément, cet exemple vise à mettre en évidence les propriétés vasodilatatrices in vivo de compositions gazeuses constituées d'un mélange de $CO_2$ et de NO au niveau coronarien sur un modèle d'ischémie myocardique multifocale chez le rat.

L'essai a été réalisé sur des rats Wistar mâles d'un poids moyen de 260/280 g, âgés de trois mois.

Pour l'administration des compositions de l'invention, on a procédé à la mise en place de cathéters souples débouchant dans le ventricule gauche.

Les rats sont mis à jeun la veille au soir de l'expérimentation, pendant 20 heures.

Une anesthésie au Chloral est pratiquée par administration intrapéritonéale de 360 mg/kg d'anesthésique. La carotide gauche est dégagée et clampée sans lésions des plans musculaires. Un cathéter souple en silastène rempli de sérum physiologique hépariné à 2% est introduit par voie rétrograde jusqu'au ventricule cardiaque gauche (environ 5 cm de cathéter). Le cathéter est maintenu en place par deux fils de ligature et est ressorti en passant sous la peau au niveau de la partie dorsale de la tête. L'incision est refermée. L'animal se réveille de son anesthésie en 60 à 90 minutes. La perméabilité du cathéter est vérifiée et du sérum physiologique hépariné reste dans le cathéter.

Trois heures après le réveil, l'activité spontanée des animaux est redevenue normale. L'extrémité du cathéter est prolongée avec un cathéter classique (Biotrol) de 600 μ de diamètre intérieur d'une longueur de 30 cm pour les injections ultérieures.

Diverses expériences sont réalisées sur un groupe de rats sains et sur un groupe de rats embolisés.

Le protocole opératoire suivi afin d'induire l'ischémie myocardique multifocale est le suivant.

L'embolisation est provoquée par l'administration chez un rat sain de 0,4 ml de dextran à 20% contenant 40000 microsphères d'albumine de 50 μ microsonnées et maintenues en suspension par agitation. L'injection est réalisée en une minute par l'intermédiaire du cathéter intra-ventriculaire.

0,2 ml de sérum physiologique sont alors administrés par la même voie pour pousser l'ensemble des microsphères dans le ventricule. Ces microsphères se répartissent dans l'organisme en fonction des fractions de débit cardiaque et en particulier dans le myocarde. Chaque microsphère obture une artériole et entraîne une microembolisation. Après l'administration des microsphères, un laps de temps de 30 minutes est respecté pour le développement de l'ischémie avant l'apparition de l'oedème. Les rats de l'autre moitié du lot ne sont pas embolisés.

Le cathéter est alors connecté à un régulateur de débit gazeux relié à une source d'approvisionnement du gaz devant être injecté.

Du vert d'indocyanine (10 μl) est placé dans le cathéter afin de visualiser le déplacement du sérum physiologique. Le régulateur de débit gazeux est ouvert et le gaz est poussé à débit constant dans le cathéter pendant une minute.

Après ce temps d'une minute, le cathéter est déconnecté du régulateur, et le reste du gaz se trouvant dans le cathéter est poussé par 0,2 ml de sérum physiologique.

Immédiatement (10 secondes) après une solution d'isopropyl iodo amphétamine (IAMP) marquée à l'iode 125 sous un volume de 0,2 ml est administrée par la même voie et poussée par 0,2 ml de sérum physiologique. 5 minutes après cette dernière administration, les rats sont sacrifiés par décapitation. Du sang est prélevé sur héparine et rapidement centrifugé pour isoler le plasma et les hématies. Environ 200 mg de foie sont prélevés. Le coeur est dégagé et séparé en endo et épicarde par dissection.

La radioactivité des quatre tissus, plasma, foie, épi et endocarde, est alors comptée par spectrométrie gamma (Appareil Intertechnique) en réglant le canal A sur le pic de l'iode 125.

Les résultats obtenus sont exprimés en nombre de coups par minute et par gramme de tissus.

La radioactivité du plasma nous permet de calculer un débit sanguin dans chaque tissu compte-tenu du débit cardiaque moyen qui est de 180 ml/mn chez le rat.

Les débits tissulaires sont exprimés en ml/mn/100 g de tissu.

EXEMPLE 2A

Une première série d'expérience a été réalisée en suivant le protocole général exposé ci-dessus à partir de 12 rats, dont 6 sains et 6 embolisés, sans injection de gaz.

Les valeurs témoins correspondantes des débits sanguins ont été rapportées dans le tableau A ci-dessous:

## TABLEAU A

| | Débits sanguins (ml/mn/100 g) | |
|---|---|---|
| | Rats témoins | Rats embolisés |
| Endocarde | 28,7 +/-1,4 | 20,5 +/-1,1** |
| Epicarde | 24,6 +/-1,1 | 23,3 +/-0,8 |
| R = Endo/Epi | 1,16 +/-0,15 | 0,87 +/-0,02** |
| Foie | 73 +/-3 | 68 +/-3 |

n = 6, m +/- ESM, ** p = 0,01 (test t de Student) comparaison entre embolisés ou non.

Le débit basal de l'endocarde est supérieur à celui de l'épicarde d'où un rapport endocarde sur épicarde supérieur à 1. L'embolisation porte plus sur l'endocarde que sur l'épicarde d'où une inversion significative du rapport. L'embolisation entraine une diminution significative des débits cardiaques alors qu'au niveau du foie, organe pris comme référence, la diminution observée est très faible.

EXEMPLE 2B

Une deuxième série d'expériences a été réalisée en suivant le protocole général exposé à l'exemple 2 à partir d'un groupe de 6 rats sains auxquels on administre des mélanges de NO et $CO_2$.

Le NO a été perfusé pendant une minute aux doses de 20 ppm avec un débit de 0,4; 0,6 respectivement 0,8 ml/mn et de 200 ppm avec un débit de 0,6 ml/mn.

Le tableau B ci-dessus rapporte l'ensemble des résultats obtenus :

TABLEAU B

| Débits sanguins (ml/mn/100 g) | | | | |
|---|---|---|---|---|
| | C1=20 ppm d2=0,4 ml/mn | C1=20 ppm d2=0,6 ml/mn | C1=20 ppm d2=0,8 ml/mn | C1=200 ppm d2=0,6 ml/mn |
| Endocarde | 44,8 +/-1,0 | 51,2 =/-1,2 | 55,8 +/-1,4 | 34,5 +/-1,4 |
| Epicarde | 48,2 +/-1,5 | 57,8 +/-1,7 | 64,2 +/-1,2 | 36,7 +/-1,6 |
| R=Endo/Epi | 0,93+/-0,02 | 0,89+/-0,01 | 0,87+/-0,01 | 0,94+/-0,01 |
| Foie | 79+/-4 | 78+/-4 | 74+/-2 | 66+/- 6 |

n = 6, m+/-ESM

1 : C représente la concentration de NO dans le gaz administré.
2 : d représente le débit: du gaz au cours de l'injection.

Les débits au niveau de l'endocarde et de l'épicarde sont significativement augmentés par les faibles doses de NO.

**[0068]** La Figure 1 annexée, tracée à partir des résultats obtenus aux exemples 2A et 2B, rapporte les variations des débits sanguins au niveau de l'endocarde et de l'épicarde en fonction du débit de perfusion chez des rats sains. Il résulte clairement de cette courbe que le débit sanguin tissulaire augmente proportionnellement au débit de perfusion, c'est-à-dire à la quantité totale de NO injectée.

Néanmoins, on constate une nette diminution des débits sanguins avec une augmentation importante (d'un facteur 10) de la concentration en NO dans le gaz administré.

EXEMPLE 2C

Une troisième série d'expériences a été réalisée en suivant le protocole général exposé à l'exemple 2 à partir d'un groupe de 6 rats embolisés auxquels on administre des mélanges de NO et $CO_2$.

Le NO a été perfusé pendant une minute aux doses de 20 ppm avec un débit de 0,4; 0,6; respectivement 0,8 ml/mn et de 200 ppm avec un débit de 0,6 ml/mn.

TABLEAU C

| Débits sanguins (ml/mn/100 g) | | | | |
|---|---|---|---|---|
| | C1=20 ppm d2=0,4 ml/mn | C1=20 ppm d2=0,6 ml/mn | C1=20 ppm d2=0,8 ml/mn | C1=200 ppm d2=0,6 ml/mn |
| Endocarde | 42,2 +/-1,3 | 46,2 =/-1,5 | 51,8 +/-1,5 | 38,0 +/-1,8 |
| Epicarde | 41,2 +/-1,4 | 43,2 +/-1,0 | 44,2 +/-1,3 | 38,8 +/-2,0 |
| R=Endo/Epi | 1,03+/-0,03 | 1,07+/-0,02 | 1,17+/-0,03 | 0,98+/-0,01 |
| Foie | 69+/-3 | 68+/-3 | 67+/-3 | 68+/-3 |

n = 6, m+/-ESM

1 : C représente la concentration de NO dans le gaz administré.
2 : d représente le débit de gaz au cours de l'injection.

En fonction de la dose administrée, le NO entraîne une augmentation du débit au niveau de l'endocarde. Dans une plus faible mesure, un effet identique est observé au niveau de l'épicarde. Ceci se traduit par un rétablissement du rapport R comme chez les témoins.

La Figure 2 rassemble les résultats obtenus à cet exemple et ceux de l'exemple 2A mettant en jeu des animaux embolisés n'ayant subi aucune administration de gaz. Plus précisément, la courbe de la Figure 2 retrace les variations des débits sanguins au niveau de l'épicarde et de l'endocarde en fonction du débit de gaz injecté.

En fait par rapport aux animaux non embolisés, le NO aux doses faibles, entraîne une redistribution des débits vers l'endocarde, région la plus ischémiée. Cet effet est associé à l'augmentation globale du débit.

Le NO redistribue donc le sang vers les régions les plus embolisées, d'où l'utilité des compositions de l'invention dans le traitement d'infarctus aigus.

Cette expérience met par ailleurs en évidence l'absence d'activité du NO administré au niveau hépatique confirmant l'action locale du monoxyde d'azote. De plus, on constate à nouveau qu'à une concentration trop élevée (200 ppm) du NO dans le gaz injecté, l'effet thérapeutique se trouve diminué.

Après chaque administration de gaz (exemples 2B et 2C), du sang de chaque rat a été prélevé. Les hématies séparées du plasma ont été hémolysées et le pourcentage de méthémoglobine par rapport à l'hémoglobine a été déterminé par spectrométrie UV sur un appareil UNICAM. Aucune modification du taux de méthémoglobine n'a pu être mise en évidence par rapport à un échantillon de sang de rat témoin.

EXEMPLE 3

Cet exemple est une étude de la toxicité éventuelle du NO gazeux administré par voie intra-cardiaque. En utilisant le même protocole d'administration qu'exposé à l'exemple 2A on a procédé aux trois expériences suivantes:

EXEMPLE 3A

Deux rats ont reçu une perfusion de NO + $CO_2$ à une concentration de 200 ppm en NO et un débit de 0,8 ml/mn pendant 15 minutes. Des troubles passagers du comportement ont été observés pendant et à la fin de la perfusion; puis les rats ont retrouvé un comportement normal (aucune mortalité après huit jours).

EXEMPLE 3B

Quatre rats ont reçu une perfusion de NO + $CO_2$ à une concentration de 200 ppm et un débit de 1,2 ml/mn pendant 15 minutes. Après des convulsions, les animaux développent une paralysie du train postérieur. Deux des 4 animaux sont morts dans l'heure qui suit la fin de la perfusion. Les deux autres survivent mais restent partiellement paralysés.

EXEMPLE 3C

Deux rats reçoivent une perfusion de $CO_2$ à un débit de 1,2 ml/mn pendant 15 minutes. Une paralysie du train postérieur est observée sans mortalité.

La perfusion de NO + $CO_2$ à une concentration de 200 ppm et un débit de 1,2 ml/mn pendant 15 minutes entraîne une mortalité de 50% des animaux alors qu'à un débit de 0,8 ml/mn il n'y a pas de mortalité. IL semble cependant que la vitesse de perfusion soit déterminante puisque le $CO_2$ se révèle presqu'aussi toxique.

Ainsi, il apparaît que les doses toxiques sont nettement plus importantes que les doses actives, ce qui permet une utilisation thérapeutique des compositions de l'invention sans risque majeur.

**Revendications**

**1.** Utilisation d'une composition gazeuse contenant du monoxyde d'azote (NO) et du dioxyde de carbone ($CO_2$) pour la fabrication d'un médicament gazeux destiné au traitement ou à la prophylaxie par voie intra-vasculaire de l'ischémie.

**2.** Utilisation d'une composition gazeuse contenant du monoxyde d'azote (NO) et du dioxyde de carbone ($CO_2$) pour la fabrication d'un médicament gazeux destiné au traitement ou à la prophylaxie par voie intravasculaire de l'embolie.

**3.** Utilisation selon l'une des revendications 1 ou 2, **caractérisée en ce que** ledit médicament gazeux comprend, en outre, au moins un gaz choisi dans le groupe formé par le protoxyde d'azote, le xénon, le krypton et leurs mélanges.

**4.** Utilisation selon l'une des revendications 1 à 3, **caractérisée en ce que** ledit médicament gazeux contient une concentration efficace de NO.

**5.** Utilisation selon l'une des revendications 1 à 4, **caractérisée en ce que** ledit médicament gazeux contient une concentration de NO comprise entre 1 et 100 ppm.

**6.** Utilisation selon l'une des revendications 1 à 5, **caractérisée en ce que** ledit médicament gazeux contient une concentration de NO comprise entre 15 et 30 ppm.

**7.** Utilisation selon l'une des revendications 1 ou 2, **caractérisée en ce que** la voie intravasculaire est la voie intra-artérielle.

**8.** Utilisation selon l'une des revendications 1 ou 2, **caractérisée en ce que** la voie intravasculaire est la voie intra-cardiaque.

**9.** Utilisation selon l'une des revendications 1 ou 2, **caractérisée en ce que** la voie intravasculaire est la voie intra-veineuse.

**Patentansprüche**

**1.** Verwendung einer Stickstoffmonoxid (NO) und Kohlendioxid ($CO_2$) enthaltenden gasförmigen Zusammensetzung zur Herstellung eines gasförmigen Arzneimittels zur Behandlung oder Prophylaxe von Ischämie auf intravaskulärem Wege.

**2.** Verwendung einer Stickstoffmonoxid (NO) und Kohlendioxid ($CO_2$) enthaltenden gasförmigen Zusammensetzung zur Herstellung eines gasförmigen Arzneimittels zur Behandlung oder Prophylaxe von Embolie auf intravaskulärem Wege.

**3.** Verwendung nach Anspruch 1 oder 2, **dadurch gekennzeichnet, daß** das gasförmige Arzneimittel außerdem auch noch mindestens ein Gas aus der Gruppe bestehend aus Distickstoffmonoxid, Xenon, Krypton und deren Gemischen enthält.

**4.** Verwendung nach einem der Ansprüche 1 bis 3, **dadurch gekennzeichnet, daß** das gasförmige Arzneimittel eine wirksame NO-Konzentration enthält.

**5.** Verwendung nach einem der Ansprüche 1 bis 4, **dadurch gekennzeichnet, daß** das gasförmige Arzneimittel eine NO-Konzentration zwischen 1 und 100 ppm enthält.

**6.** Verwendung nach einem der Ansprüche 1 bis 5, **dadurch gekennzeichnet, daß** das gasförmige Arzneimittel eine NO-Konzentration zwischen 15 und 30 ppm enthält.

**7.** Verwendung nach Anspruch 1 oder 2, **dadurch gekennzeichnet, daß** es sich bei dem intravaskulären Weg um den intraarteriellen Weg handelt.

**8.** Verwendung nach Anspruch 1 oder 2, **dadurch gekennzeichnet, daß** es sich bei dem intravaskulären Weg um den intracardialen Weg handelt.

**9.** Verwendung nach Anspruch 1 oder 2, **dadurch gekennzeichnet, daß** es sich bei dem intravaskulären Weg um den intravenösen Weg handelt.

**Claims**

**1.** Use of a gaseous composition containing nitric oxide (NO) and carbon dioxide ($CO_2$) for preparing a gaseous drug for intravascular treatment or prevention of ischaemia.

**2.** Use of a gaseous composition containing nitric oxide (NO) and carbon dioxide ($CO_2$) for preparing a gaseous drug for intravascular treatment or prevention of embolisms.

**3.** Use according to either of Claims 1 and 2, **characterized in that** said gaseous drug additionally comprises at least one gas chosen from the group of nitrogen protoxide, xenon, krypton and their mixtures.

4. Use according to one of Claims 1 to 3, **characterized in that** said gaseous drug contains an effective concentration of NO.

5. Use according to one of Claims 1 to 4, **characterized in that** said gaseous drug contains a concentration of NO of between 1 and 100 ppm.

6. Use according to one of Claims 1 to 5, **characterized in that** said gaseous drug contains a concentration of NO of between 15 and 30 ppm.

7. Use according to either of Claims 1 and 2, **characterized in that** the intravascular route is an intra-arterial route.

8. Use according to either of Claims 1 and 2, **characterized in that** the intravascular route is an intracardial route.

9. Use according to either of Claims 1 and 2, **characterized in that** the intravascular route is an intravenous route.

PERFUSION DE NO A 20 ppm

DEBIT ml/mn/100 g

DEBIT DE PERFUSION ml/mn
—•— Endocarde
—o— Epicarde

FIG.1

EP 0 855 912 B1

PERFUSION DE NO A 200ppm

DEBIT ml/mn/100g

DEBIT DE PERFUSION ml/mn

●— Endocarde
—o— Epicarde

FIG.2

EP 0 855 912 B1